(19) Europäisches Patentamt
European Patent Office
Office européen des brevets

(11) Publication number: **0 411 183 A1**

# (12) EUROPEAN PATENT APPLICATION

(21) Application number: **89114272.1**

(22) Date of filing: **02.08.89**

(51) Int. Cl.5: **C07D 407/04**, C07D 405/14, C09K 15/06, C09K 15/16, A61K 31/375

(43) Date of publication of application: **06.02.91 Bulletin 91/06**

(84) Designated Contracting States: **AT BE CH DE ES FR GB GR IT LI LU NL SE**

(71) Applicant: **NIPPON HYPOX LABORATORIES INCORPORATED**
**1759, Matsugaya Hachioji-shi**
**Tokyo 192-03(JP)**

(72) Inventor: **Satoh, Toshio**
**57-3, Nagao Jyoroku-cho**
**Tokushima-shi Tokushima-ken(JP)**
Inventor: **Niiro, Yasunori No. 403**
**2nd Masuoka Bldg. 1-3,**
**Minamisako-hachiban-cho**
**Tokushima-shi Tokushima-ken(JP)**
Inventor: **Kakegawa, Hisao**
**No. 307, City-Corpo. Kimura 3-59,**
**Okihama-higashi**
**Tokushima-shi Tokushima-ken(JP)**
Inventor: **Matsumoto, Hitoshi**
**125-22, Shimofukuman Hachiman-cho**
**Tokushima-shi Tokushima-ken(JP)**

(74) Representative: **Hansen, Bernd, Dr.rer.nat. et al**
**Hoffmann, Eitle & Partner Patentanwälte**
**Arabellastrasse 4 Postfach 81 04 20 20**
**D-8000 München 81(DE)**

## (54) Ascorbic acid derivative.

(57) An antioxidant comprising an ascorbic acid derivative represented by the general formula

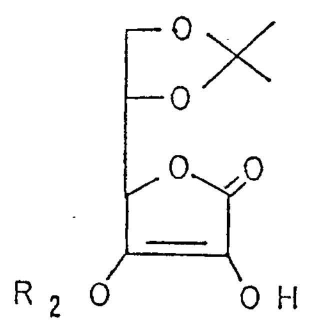

wherein $R_2$ is a group selected from the group consisting of an alkyl group, a heterocyclic ring-containing alkyl group, an aralkyl group, a hydroxycarbonyl-alkyl group, an alkoxycarbonyl-alkyl group, an aral-koxycarbonyl-alkyl group, an alkenyl group, a hydroxy-alkyl group, an alkoxy-alkyl group, an alkylcarbonyl-alkyl group, an arylcarbonyl-alkyl group and a cyano-alkyl group.

Compounds of the same formula in which $R_1$ is selected from the group consisting of a heterocyclic ring-containing alkyl group, an alkylcarbonyl-alkyl group and an arylcarbonyl-alkyl group are new.

EP 0 411 183 A1

# ASCORBIC ACID DERIVATIVE

## BACKGROUND OF THE INVENTION

### (1) Field of the Invention

This invention relates to an ascorbic acid derivative, a process for preparing the same and an antioxidant comprising an ascorbic acid derivative.

### (2) Prior Art

Ascorbic acid has antioxidant action and is used for the purpose of preventing browning of foods, retaining flavor or freshness of foods or the like.

Ascorbic acid, however, is susceptible to decomposition and sometimes hard to produce the above-mentioned effects over a long period.

## SUMMARY OF THE INVENTION

It is, therefore, a first object of this invention to provide a novel ascorbic acid derivative eliminating the aforementioned disadvantages of the ascorbic acid. It is a second object of this invention to provide a process for preparing the aforesaid novel ascorbic acid derivative. It is further a third object of this invention to provide an antioxidant comprising an ascorbic acid derivative.

The first object of this invention has been achieved by an ascorbic acid derivative represented by the general formula (Ia):

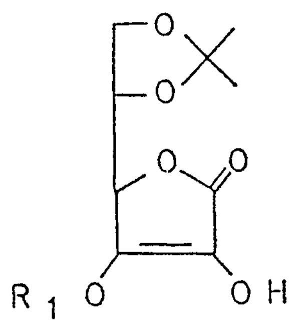

(Ia)

wherein $R_1$ is a group selected from the group consisting of a heterocyclic ring-containing $(C_1\text{-}C_5)$alkyl group, a $(C_1\text{-}C_5)$alkylcarbonyl-$(C_1\text{-}C_5)$alkyl group and a $(C_6\text{-}C_{12})$arylcarbonyl-$(C_1\text{-}C_5)$alkyl group.

The second object of this invention has been accomplished by a process for preparing an ascorbic acid derivative represented by the general formula (Ia):

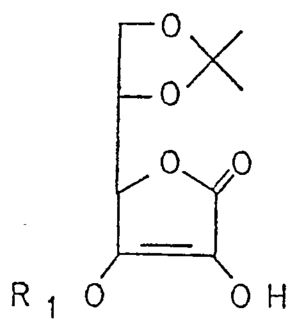

(Ia)

wherein $R_1$ is a group selected from the group consisting of a heterocyclic ring-containing ($C_1$-$C_5$)alkyl group, a ($C_1$-$C_5$)alkylcarbonyl-($C_1$-$C_5$)alkyl group and a ($C_6$-$C_{12}$)arylcarbonyl-($C_1$-$C_5$)alkyl group, comprising reacting 5,6-O-isopropylidene ascorbic acid represented by the formula (II):

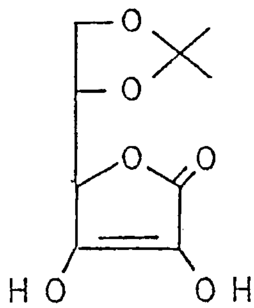

( II )

with an organic halide selected from the group consisting of a heterocyclic ring-containing ($C_1$-$C_5$)alkyl halide, a ($C_1$-$C_5$)alkylcarbonyl-($C_1$-$C_5$)alkyl halide and a ($C_6$-$C_{12}$)arylcarbonyl-($C_1$-$C_5$)alkyl halide.

Furthermore, the third object of this invention has been achieved by an antioxidant comprising an ascorbic acid derivative represented by the general formula (IA):

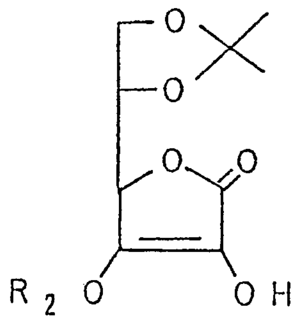

( I A )

wherein $R_2$ is a group selected from the group consisting of a ($C_1$-$C_{22}$)alkyl group, a heterocyclic ring-containing ($C_1$-$C_5$)alkyl group, a ($C_7$-$C_{12}$)aralkyl group, a hydroxycarbonyl-($C_1$-$C_{22}$)alkyl group, a ($C_1$-$C_{22}$)-alkoxycarbonyl-($C_1$-$C_5$)alkyl group, a ($C_7$-$C_{12}$)aralkoxycarbonyl-($C_1$-$C_5$)alkyl group, a ($C_2$-$C_6$)alkenyl group, a hydroxy-($C_1$-$C_5$)alkyl group, a ($C_1$-$C_5$)-alkoxy-($C_1$-$C_5$)alkyl group, a ($C_1$-$C_5$)alkylcarbonyl-($C_1$-$C_5$)alkyl group, a ($C_6$-$C_{12}$)arylcarbonyl-($C_1$-$C_5$)alkyl group and a cyano-($C_1$-$C_5$)alkyl group.

The group $R_1$ in the general formula (Ia) representing the novel ascorbic acid derivative of this invention is different in the number of substituent groups defined therein from the group $R_2$ in the general formula (IA) representing the ascorbic acid derivative constituting the antioxidant of this invention. Although the group $R_1$ has three substituent groups, the group $R_2$ has the total 12 substituent groups including the three. This means that ascorbic acid derivatives which are not novel can also be used as the antioxidant of this invention.

## DETAILED DESCRIPTION OF THE INVENTION

The novel ascorbic acid derivative of this invention is initially explained hereinafter.

The novel ascorbic acid derivative of this invention is represented by the general formula (Ia):

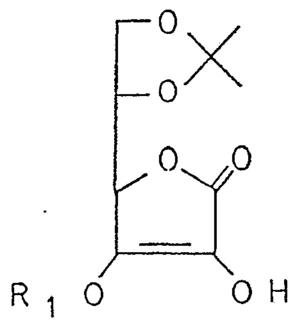

( I a )

wherein $R_1$ is a group selected from the group consisting of a heterocyclic ring-containing ($C_1$-$C_5$)alkyl group, a ($C_1$-$C_5$)alkylcarbonyl-($C_1$-$C_5$)alkyl group and a ($C_6$-$C_{12}$)arylcarbonyl-($C_1$-$C_5$)alkyl group.

Heterocyclic ring-containing ($C_1$-$C_5$)alkyl groups having 1 to 3 nitrogen atoms in the ring are herein preferred as the heterocyclic ring-containing alkyl groups, and examples thereof include groups represented by the general formulae:

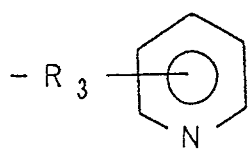

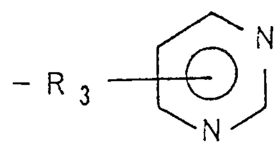

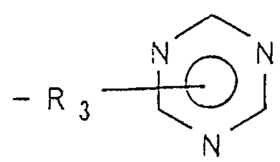

wherein $R_3$ is a ($C_1$-$C_5$)alkylene group which may optionally have a branched chain. Particularly preferred heterocyclic ring-containing alkyl groups are pyridylmethyl group, pyrimidylmethyl group, triazylmethyl group and the like.

Examples of the ($C_1$-$C_5$)alkylcarbonyl-($C_1$-$C_5$)alkyl groups include groups represented by the general formula:

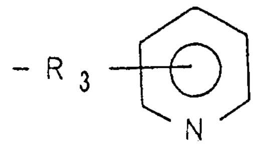

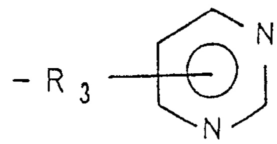

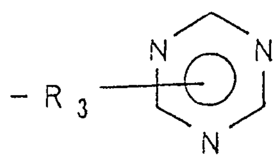

wherein $R_4$ is a ($C_1$-$C_5$)alkylene group which may optionally have a branched chain; $R_5$ is a ($C_1$-$C_5$)-alkyl group which may optionally have a branched chain. Particularly preferred alkylcarbonylalkyl groups are
$-CH_2-CO-CH_3$, $-CH_2-CH-C_2H_5$
and the like.

Furthermore, examples of the ($C_6$-$C_{12}$)arylcarbonyl-($C_1$-$C_5$)alkyl groups include groups represented by the general formula:

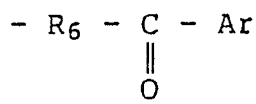

wherein $R_6$ is a ($C_1$-$C_5$)alkylene group which may optionally have a branched chain; Ar is a ($C_6$-$C_{12}$)aryl group which may optionally have a substituent group on the ring. Particularly preferred arylcarbonylalkyl groups are $-CH_2-CO-C_6H_5$ and the like.

The novel ascorbic acid derivative of this invention has excellent antioxidant action and can be preferably used as food antioxidants or beautifying and whitening cosmetics.

The novel ascorbic acid derivative of this invention is an extremely specific compound in that it has the ability to eliminate superoxides having the possibility of damaging biomolecules and tissues, and application as a medicine is also considered for treating diseases derived from such superoxides.

The process for preparing the novel ascorbic acid derivative represented by the above-mentioned general formula (Ia) of this invention is explained hereinafter.

In the process of this invention, 5,6-O-isopropylidene ascorbic acid represented by the formula (II):

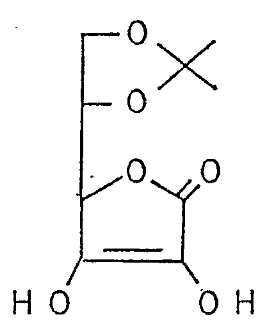

( II )

is used as a starting material.

Such a compound represented by the formula (II) is obtained by ketalizing ascorbic acid according to a conventional method.

In the process of this invention, the compound represented by the general formula (II) is reacted with an organic halide selected from the group consisting of a heterocyclic ring-containing ($C_1$-$C_5$)alkyl halide, a

$(C_1$-$C_5)$alkylcarbonyl-$(C_1$-$C_5)$alkyl halide and a $(C_6$-$C_{12})$arylcarbonyl-$(C_1$-$C_5)$alkyl halide to etherify the hydroxyl group at the 3-position of the compound represented by the general formula (II) and thereby provide the desired compound represented by the general formula (Ia). The dehydrohalogenation may be carried out according to a method wherein the compound represented by the general formula (II) is mixed under stirring with an organic halide in an organic solvent such as DMF, DMSO, THF, hexamethylphosphoramide or the like. It may be conducted by vigorously stirring the compound represented by the general formula (II) and an organic halide in the presence of a quaternary ammonium salt-phase transfer catalyst such as tetrabutylammonium bromide, N-acetonylpridinium bromide, allyltriethylammonium bromide, N-aminopyridinium iodide or the like, in a reaction system comprising an organic solvent phase (a non-aqueous solvent, such as methyl ethyl ketone, benzene, methylene chloride, chloroform or the like) and an aqueous phase (water). The above-mentioned organic solvent such as DMF, DMSO, THF, hexamethylphosphoramide or the like may be used as the organic solvent, depending on the kind of the aforesaid phase transfer catalyst.

The former method is suitable for using alkylcarbonylalkyl halides or arylcarbonylalkyl halides as the organic halide, and the latter method is suitable for using the above-mentioned two kinds of organic halides and also for using heterocyclic ring-containing alkyl halides.

The antioxidant of this invention is explained hereinafter.

As explained above, the antioxidant of this invention comprises the ascorbic acid derivative represented by the general formula (IA):

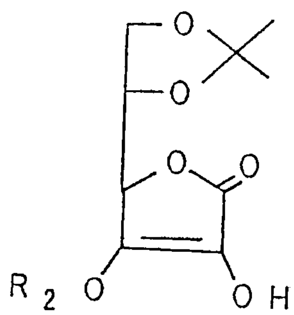

( I A )

The group $R_2$ in the formula is within a wider scope than the group $R_1$ in the general formula (Ia) representing the novel ascorbic acid derivative as described above, and therefore the ascorbic acid derivative represented by the general formula (IA) includes also known compounds.

Thus, the group $R_2$ includes the same three substituent groups (heterocyclic ring-containing alkyl group, alkylcarbonylalkyl group and arylcarbonylalkyl group) as the group $R_1$, besides those, further nine substituent groups of a $(C_1$-$C_{22})$alkyl group, a $(C_7$-$C_{12})$aralkyl group, a hydroxycarbonyl-$(C_1$-$C_{22})$alkyl group, a $(C_1$-$C_{22})$alkoxycarbonyl-$(C_1$-$C_5)$alkyl group, a $(C_7$-$C_{12})$aralkoxycarbonyl-$(C_1$-$C_5)$alkyl group, a $(C_2$-$C_6)$-alkenyl group, a hydroxy-$(C_1$-$C_5)$alkyl group, a $(C_1$-$C_5)$alkoxy-$(C_1$-$C_5)$alkyl group and a cyano-$(C_1$-$C_5)$alkyl group. The term "alkyl" in the aforesaid substituent groups means a straight or branched alkyl group. The term "alkoxy" means an alkoxy group consisting of a straight or branched alkyl group and oxygen. The term "alkenyl" means a straight or branched alkenyl group having at least one unsaturated double bond.

Such an ascorbic acid derivative represented by the general formula (IA) has a radical eliminating ability and is preferably used as an antioxidant. The ascorbic acid derivative wherein $R_2$ is an alkylcarbonylalkyl group is extremely desirable, since it has the ability to eliminate superoxides. The ascorbic acid derivative of this invention is also excellent in stability with time.

[Examples]

Examples of this invention are illustrated hereinafter.

Example 1 [Preparation of novel ascorbic acid derivative (Ia) ($R_1$ is alkylcarbonylalkyl)]

(1) Synthesis of L-5,6-O-isopropylideneascorbic acid which is a starting material

Ascorbic acid in an amount of 180 g was stirred in 750 mℓ of acetone and warmed to 40° C. Acetyl chloride in a volume of 20 mℓ was added, and stirring was continued to form a slurry layer.

After 3 hours, the slurry layer was cooled with ice to collect deposited precipitates by filtration. The resulting precipitates were washed with a mixture of cold acetone-n-hexane (3:7) on a funnel and dried with silica gel under reduced pressure.

Recrystallization from acetone was then carried out to provide 190 g of L-5,6-O-isopropylidene-ascorbic acid (melting point: 206-208° C).

(2) Synthesis of L-5,6-O-isopropylidene-3-O-(3-butanone)ascorbic acid

In 30 mℓ of dimethyl sulfoxide (DMSO), was dissolved 4.32 g of the compound obtained in (1). Sodium hydrogencarbonate in an amount of 1.66 g was added, and the resulting solution was stirred. After 30 minutes, 4 g of 2-butanone bromide was added, and the obtained solution was warmed to 50° C. After warming the solution for 20 hours, distilled water (100 mℓ) and ethyl acetate (100 mℓ x 2) were added to shake the reaction solution. Organic layers were combined, washed with water, shaken with saturated sodium chloride solution and dried over sodium sulfate. An oily substance obtained by concentrating under reduced pressure was subjected to silica gel chromatography and eluted with benzene-ethyl acetate to provide L-5,6-O-isopropylidene-3-O-(3-butanone) ascorbic acid (corresponding to compound No. 022 in Table-1 mentioned below) which was an oily substance. The resulting compound No. 022 of this invention was subjected to antioxidant tests described below.

Furthermore, the method in (2) of Example 1 could be applied to preparation of L-5,6-isopropylidene- 3-O-(propanone) ascorbic acid (corresponding to compound No. 021 in Table-1 mentioned below).

Example 2 [Preparation of novel ascorbic acid derivative (Ia) ($R_1$ is alkylcarbonylalkyl)]

The L-5,6-O-isopropylideneascorbic acid obtained in (1) of Example 1 was used as a starting material to carry out the reaction of the above-mentioned starting material with an organic halide in the presence of a phase transfer catalyst in the reaction system consisting of an organic phase and aqueous phase. Details of the reaction are as follows. Thus, 4.15 g of sodium hydrogencarbonate was dissolved in 100 mℓ of distilled water, and 200 mℓ of methyl ethyl ketone and 10.80 g of the aforementioned starting material were added. To the resulting mixture solution, 9.25 g of chloroacetone as an organic halide and 0.80 g of tetrabutylammonium bromide as a phase transfer catalyst were added. The obtained mixture was heated to 60° C and vigorously stirred. After 16 hours, the aqueous layer was adjusted to pH 4 to 5 with 4N hyrochloric acid to separate and collect the organic layer. The aqueous layer was shaken with 200 mℓ of ethyl acetate, and the resulting organic layer was combined with the aforesaid organic layer, washed with water and then dried over sodium sulfate. Isopropyl ether and petroleum ether were successively added to an oily substance obtained under reduced pressure to collect deposited precipitates by filtration. The obtained precipitates were recrystallized from ethyl acetate saturated with water to afford L-5,6-O-isopropylidene-3-O-methylcarbomethylascorbic acid (corresponding to compound No. 021 in Table-1 mentioned below) having the melting point of 71° C. The resulting compound No. 021 of this invention was used for the antioxidant tests described below.

Example 3 [Preparation of novel ascorbic acid derivative (Ia) ($R_1$ is 2′-pyridylmethyl)]

The procedures similar to those in Example 1 were carried out, except that 2′-pyridylmethyl bromide was used as an organic halide in place of chloroacetone and various reaction conditions were modified according to change of the organic halide to obtain L-5,6-O-isopropylidene-3-O-2′-pyridylmethylascorbic acid (corresponding to compound No. 007 in Table-1 mentioned below) having the melting point of 144 to 145° C. The compound No. 007 of this invention was used for the antioxidant tests mentioned below.

Referential example 1

[Preparation of other ascorbic acid derivatives included in the ascorbic acid derivative (IA)]

The L-5,6-O-isopropylideneascorbic acid obtained in (1) of Example 1 was used as a starting material, and reaction was carried out according to the method described in (2) of Example 1 to provide 22 ascorbic acid derviatives designated as compound Nos. 001, 002, 003, 004, 005, 006, 008, 009, 010, 011, 012, 013, 014, 015, 016, 017, 018, 019, 020, 023, 024 and 025 in Table-1 mentioned below. The resulting ascorbic acid derivatives were used for the antioxidant tests described below.

The compound Nos. 003 an 006 could be synthesized by the method mentioned in Example 2.

Table-1

| Compound No. | $R_1$ | mp | NMR δ value |
|---|---|---|---|
| 001 | $-(CH_2)_{17}CH_3$ | 41.5-43°C | 0.88 (3H, m) 1.29 (32H, 6Hm) 4.15 (3H, m) 4.49 (2H, t) 4.67 (H, d, 3Hz) |
| 002 | $-(CH_2)_{11}CH_3$ | Oily substance | 0.88 (3H, m) 1.30 (20H, 6Hm) 4.15 (3H, m) 4.49 (2H, t) 4.67 (H, d, 3Hz) |
| 003 | $-(CH_2)_7CH_3$ | Oily substance | 0.89 (3H, m) 1.29 (12H, 6Hm) 4.15 (3H, m) 4.49 (2H, t) 4.67 (H, d, 3Hz) |
| 004 | $-(CH_2)_3CH_3$ | Oily substance | 0.94 (3H, m) 1.29 (4Hm) 1.34 (3H, s), 1.35 (3H, s) 4.15 (3H, m) 4.48 (2H, t) 4.67 (H, d, 3Hz) |
| 005 | $-CH(CH_3)-CH_2CH_3$ | Oily substance | 0.97 (3H, t) 1.30 (6Hs) 1.37 (3H, d) 1.65 (2H, m) 4.16 (3H, m) 4.65 (H, d, d) 4.95 (H, m) |
| 006 | $-CH_2-$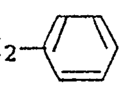 | 105-106°C | 1.30 (6H, s) 4.15 (3H, m) 4.66 (H, d, 3Hz) 5.49 (2H, s) 7.37 (5H, bs) |
| 007 | $-CH_2-$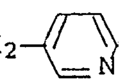 | 144-145°C | 1.29 (6H, s) 4.14 (3H, m) 4.72 (H, d, 3Hz) 5.59 (2H, s) 7.47 (H, m) 7.97 (H, m) 8.53 (H, m) 8.63 (H, m) |
| 008 | $-(CH_2)_4COOC_2H_5$ | Oily substance | 1.21 (3H, t) 1.29 (6H, s) 1.77 (2H, m) 2.37 (2H, m) 4.09 (2H, q) 4.16 (3H, m) 4.51 (2H, m) 4.69 (H, d, 3Hz) |
| 009 | $-(CH_2)_{16}COOH$ | Oily substance | 1.30 (16H, m) 1.37 (3H, s) 1.40 (3H, s) 2.36 (2H, t) 4.15 (3H, m) 4.49 (2H, t) 4.57 (H, d, 3Hz) |

- Cont'd -

Table-1 (Cont'd)

| Compound No. | $R_1$ | mp | NMR δ value |
|---|---|---|---|
| 010 | $-(CH_2)_3COOC_2H_5$ | Oily substance | 1.22 (3H, t) 1.29 (6H, s) 2.17 (2H, m) 2.47 (2H, m) 4.10 (2H, q) 4.15 (3H, m) 4.53 (2H, t) 4.70 (H, d, 3Hz) |
| 011 | $-CH_2CH=CH_2$ | Oily substance | 1.29 (6H, s) 4.15 (3H, m) 4.71 (H, d, 3Hz) 4.98 (2H, m) 5.28 (H, m) 5.43 (H, m) 6.10 (H, m) |
| 012 | $-CH_2CH_2OH$ | Oily substance | 1.30 (6H, s) 3.83 (2H, t) 4.15 (3H, m) 4.52 (2H, t, H, s) 4.68 (H, d, 3Hz) |
| 013 | $-CH_2OCH_3$ | 92°C | 1.29 (6H, s) 3.51 (3H, s) 4.15 (3H, m) 4.77 (H, d, 3Hz) 5.46 (2H, s) |
| 014 | $CH_3$<br>$-CH-COOC_2H_5$ | Oily substance | 1.26 (3H, d, t) 1.30 (6H, s) 1.55 (3H, dd) 4.15 (5H, m) 4.75 (H, d, 3Hz) 5.40 (H, m) |
| 015 | $-CH_2COOH$ | Oily substance | 1.31 (6H, s) 4.15 (3H, m) 4.76 (H, d, 3Hz) 5.05 (2H, d, 2Hz) |
| 016 | $-CH_2COOCH_2-$ 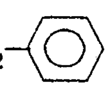 | Oily substance | 1.29 (3H, s) 1.30 (3H, s) 4.15 (3H, m) 4.75 (H, d, 3Hz) 5.11 (2H, s) 5.25 (2H, s) 7.36 (5H, s) |
| 017 | $-CH_2COOC(CH_3)_3$ | 116-122°C | 1.29 (3H, s) 1.44 (9H, s) 4.15 (3H, m) 4.70 (H, d, 3Hz) 4.84 (2H, s) |

- Cont'd -

Table-1 (Cont'd)

| Compound No. | $R_1$ | mp | NMR δ value |
|---|---|---|---|
| 018 | $-CH_2COO-n-C_{10}H_{21}$ | 52-54°C | 0.88 (3H, m) 1.30 (6H, 16H, m) 4.16 (5H, m) 4.74 (H, d, 3Hz) 5.03 (2H, s) |
| 019 | $-CH_2COOC_4H_9$ | Oily substance | 0.91 (3H, m) 1.31 (6H, s) 1.56 (4H, m) 4.14 (5H, m) 4.76 (H, d, 3Hz) 5.04 (2H, s) |
| 020 | $-CH_2COOC_2H_5$ | 94°C | 1.26 (3H, t) 1.31 (6H, s) 4.14 (5H, m) 4.76 (H, d, 3Hz) 5.03 (2H, s) |
| 021 | $-CH_2COCH_3$ | 71°C | 1.27 (6H, s) 2.09 (3H, s) 4.16 (3H, m) 4.86 (H, d, 3Hz) 5.03 (2H, s) |
| 022 | $-CH_2COC_2H_5$ | Oily substance | 1.04 (3H, t) 1.31 (6H, s) 2.55 (2H, q) 4.15 (3H, m) 4.77 (H, d, s) 5.10 (2H, s) |
| 023 | $-CH_2CO$ 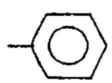 | 175°C | 1.33 (6H, s) 4.20 (3H, m) 4.84 (H, d, 4Hz) 5.85 (2H, d, 2Hz) 7.62 (3H, m) 8.01 (2H, m) |
| 024 | $-CH_2CN$ | Oily substance | 1.29 (6H, s) 4.14 (3H, m) 4.84 (H, d, 3Hz) 5.36 (2H, s) |
| 025 | $-CH_2CH_3$ | 105°C | 1.28 (6H, s) 1.34 (3H, t) 4.12 (3H, m) 4.51 (2H, q) 4.65 (H, d, 3Hz) |

Test example 1 (Antioxidant action examined by using stable radicals)

Reduction activity of α,-α-diphenyl-β-picrylhydrazyl (DPPH) which was a stable free radical was examined according to the M.S. Blois method (Nature, vol. 181, page. 1199, 1958) and used as an index to antioxidant action. Thus, specimens were added to 3 mℓ of a 0.1 mM DPPH solution in ethanol, and absorbance at a wavelength of 517 nm was measured using a spectrophotometer after 20 minutes. The difference in absorbance from the solvent control [0.5% or less of dimethylformamide (DMF)] was taken as the reduction activity.

The 50% radical eliminating concentrations for the test compounds are shown in Table-2.

As can be seen from Table-2, the tested compounds of this invention were found to have improved antioxidant action.

Table-2

| Compound No. | 50% radical eliminating concentration |
|---|---|
| 001 | 3.0 x $10^{-5}$ M |
| 002 | 2.9 |
| 003 | 2.7 |
| 004 | 2.5 |
| 005 | 3.2 |
| 006 | 2.2 |
| 007 | 2.3 |
| 008 | 1.6 |
| 009 | 2.5 |
| 010 | 1.6 |
| 011 | 3.3 |
| 012 | 3.5 |
| 013 | 1.7 |
| 014 | 2.6 |
| 015 | 3.1 |
| 016 | 2.5 |
| 017 | 2.5 |
| 018 | 2.8 |
| 019 | 2.5 |
| 020 | 2.3 |
| 021 | 1.7 |
| 022 | 2.0 |
| 023 | 2.4 |
| 024 | 3.1 |
| 025 | 2.2 |

Test example 2 (Effects on formazan formation in the xanthine-xanthine oxidase-Nitrotetrazolium Blue system)

Specimens were added to a 0.05 M phosphate buffer solution (pH 7.8) containing xanthine, EDTA 2Na and Nitrotetrazolium Blue (NTB) so as to provide the respective final concentrations of $5x10^{-5}$ M, $1x10^{-4}$ M and $5x10^{-4}$ M and warmed at 30°C for 3 minutes. Xanthine oxidase (manufactured by Sigma Corp.) was added, and the resulting specimens were warmed at 30°C for 10 minutes to colorimetrically determine formazan formed by superoxide anion radicals. The inhibition ratio of formazan formation was determined

from the results at concentrations of 0.5 mM and 4 mM of each specimen.

Results are shown in Table-3. As can be seen from Table-3, tested compound Nos. 021 and 022 of this invention remarkably inhibited formazan formation and therefore the compound of this invention has excellent ability to eliminate superoxide anion radicals.

Table-3

| Compound | Concentration (mM) | Inhibition ratio (%) of formazan formation |
|---|---|---|
| 021 | 0.5 | 9.2 |
| | 4 | 47.4 |
| 022 | 0.5 | 22.6 |
| | 4 | 26.7 |

As detailed above, this invention provides a novel ascorbic acid derivative having excellent antioxidant action, particularly eliminating action on superoxides and a process for preparing the same.

Furthermore, this invention also provides a novel antioxidant comprising the afore-mentioned novel ascorbic acid derivative or other known ascorbic acid derivatives.

## Claims

1. An ascorbic acid derivative represented by general formula (Ia):

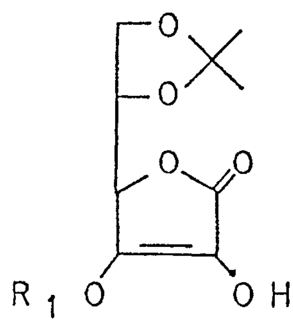

(Ia)

wherein $R_1$ is selected from the group consisting of a heterocyclic ring-containing $(C_1-C_5)$alkyl group, a $(C_1-C_5)$alkylcarbonyl-$(C_1-C_5)$alkyl group and a $(C_6-C_{12})$arylcarbonyl-$(C_1-C_5)$alkyl group.

2. An ascorbic acid derivative of Claim 1, wherein said heterocyclic ring-containing $(C_1-C_5)$alkyl group is a heterocyclic ring-containing $(C_1-C_5)$alkyl group containing 1 to 3 nitrogen atoms in the heterocyclic ring.

3. An ascorbic acid derivative of Claim 2, wherein said heterocyclic ring-containing $(C_1-C_5)$alkyl group is represented by general formula:

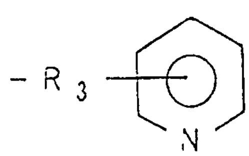

,

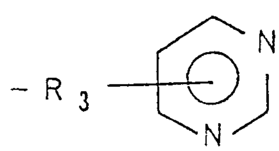

or

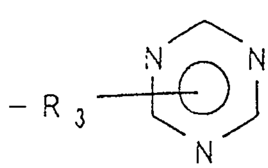

wherein $R_3$ represents a $(C_1-C_5)$alkylene group which may optionally have a branched chain.

4. An ascorbic acid derivative of Claim 3, wherein said heterocyclic ring-containing $(C_1-C_5)$alkyl group is selected from the group consisting of pyridylmethyl, pyrimidylmethyl and triazylmethyl.

5. An ascorbic acid derivative of Claim 1, wherein said alkylcarbonylalkyl group is represented by general

formula:

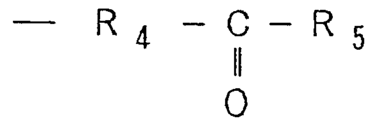

wherein $R_4$ represents a $(C_1-C_5)$alkylene group which may optionally have a branched chain and $R_5$ represents a $(C_1-C_5)$alkyl group which may optionally have a branched chain.

6. An ascorbic acid derivative of Claim 5, wherein said $(C_1-C_5)$alkylcarbonyl-$(C_1-C_5)$alkyl group is selected from the group consisting of $-CH_2-CO-CH_3$ and $-CH_2-CO-C_2H_5$

7. An ascorbic acid derivative of Claim 1, wherein said $(C_6-C_{12})$arylcarbonyl-$(C_1-C_5)$alkyl group is represented by general formula:

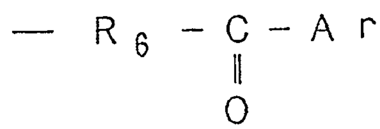

wherein $R_6$ represents a $(C_1-C_5)$alkylene group which may optionally have a branched chain and Ar is a $(C_6-C_{12})$aryl group which may optionally have a substituent thereon.

8. An ascorbic acid derivative of Claim 7, wherein said $(C_6-C_{12})$arylcarbonyl-$(C_1-C_5)$alkyl group is $-CH_2-CO-C_6H_5$

9. A process for preparing an ascorbic acid derivative represented by general formula (Ia):

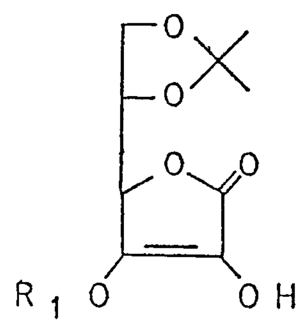

(Ia)

wherein $R_1$ is a group selected from the group consisting of a heterocyclic ring-containing $(C_1-C_5)$alkyl group, a $(C_1-C_5)$alkylcarbonyl-$(C_1-C_5)$alkyl group and a $(C_6-C_{12})$arylcarbonyl-$(C_1-C_5)$alkyl group, which comprises reacting 5,6-O-isopropylideneascorbic acid represented by general formula (II):

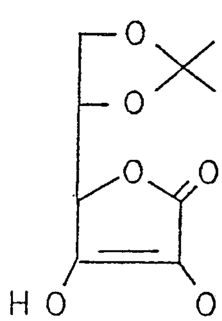

(II)

with an organic halide selected from the group consisting of a heterocyclic ring-containing $(C_1-C_5)$alkyl halide, a $(C_1-C_5)$alkylcarbonyl-$(C_1-C_5)$alkyl halide and a $(C_6-C_{12})$arylcarbonyl-$(C_1-C_5)$alkyl halide.

10. A process of Claim 9, wherein said reaction is carried out in the presence of a solvent selected from the group consisting of DMF, DMSO, THF and hexamethylphosphoramide.

11. A process of Claim 9, wherein said reaction is carried out in a reaction system comprising a non-aqueous solvent and water in the presence of a phase transfer catalyst.

12. A process of Claim 11, wherein said catalyst is a quaternary ammonium salt selected from the group consisting of tetrabutylammonium bromide, N-acetonylpyridinium bromide, allyltriethylammonium bromide and N-aminopyridinium iodide.

13. An antioxidant comprising an ascorbic acid derivative represented by the general formula (IA):

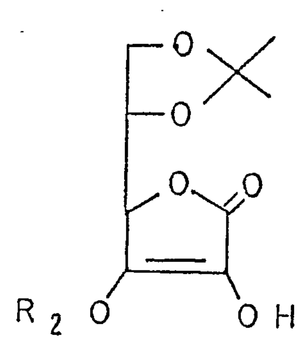

( I A )

wherein $R_2$ is a group selected from the group consisting of a ($C_1$-$C_{22}$)alkyl group, a heterocyclic ring-containing ($C_1$-$C_5$)alkyl group, a ($C_7$-$C_{12}$)aralkyl group, a hydroxycarbonyl-($C_1$-$C_{22}$)alkyl group, a ($C_1$-$C_{22}$)-alkoxycarbonyl-($C_1$-$C_5$)alkyl group, a ($C_7$-$C_{12}$)aralkoxycarbonyl-($C_1$-$C_5$)alkyl group, a ($C_2$-$C_6$)alkenyl group, a hydroxy-($C_1$-$C_5$)alkyl group, a ($C_1$-$C_5$)-alkoxy-($C_1$-$C_5$)alkyl group, a ($C_1$-$C_5$)alkylcarbonyl- ($C_1$-$C_5$)alkyl group, a ($C_6$-$C_{12}$)arylcarbonyl-($C_1$-$C_5$)alkyl group and a cyano-($C_1$-$C_5$)alkyl group.

14. An antioxidant of Claim 13, wherein said heterocyclic ring-containing ($C_1$-$C_5$)alkyl group is a heterocyclic ring-containing ($C_1$-$C_5$)alkyl group containing 1 to 3 nitrogen atoms in the heterocyclic ring.

15. An antioxidant of Claim 14, wherein said heterocyclic ring-containing ($C_1$-$C_5$)alkyl group is represented by general formula:

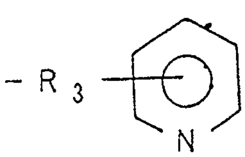 , 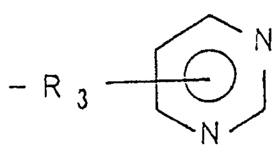 or 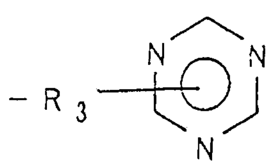

wherein $R_3$ represents a ($C_1$-$C_5$)alkylene group which may optionally have a branched chain.

16. An antioxidant of Claim 13, wherein said heterocyclic ring-containing ($C_1$-$C_5$)alkyl group is selected from the group consisting of pyridylmethyl, pyrimidylmethyl and triazylmethyl.

17. An antioxidant of Claim 13, wherein said ($C_1$-$C_5$)alkylcarbonyl-($C_1$-$C_5$)alkyl group is represented by general formula:

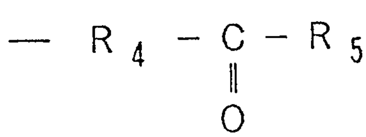

wherein $R_4$ represents a ($C_1$-$C_5$)alkylene group which may optionally have a branched chain and $R_5$ represents a ($C_1$-$C_5$)alkyl group which may optionally have a branched chain.

18. An antioxidant of Claim 17, wherein said ($C_1$-$C_5$)alkylcarbonyl-($C_1$-$C_5$)alkyl group is selected from the group consisting of $-CH_2-CO-CH_3$ and $-CO_2-CO-C_2H_5$.

19. An antioxidant of Claim 13, wherein said ($C_6$-$C_{12}$)arylcarbonyl-($C_1$-$C_5$)alkyl group is represented by general formula:

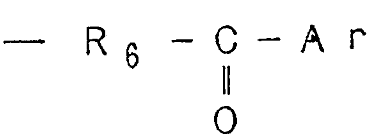

wherein $R_6$ represents a $(C_1-C_5)$alkylene group which may optionally have a branched chain and Ar is a $(C_6-C_{12})$aryl group which may optionally have a substituent thereon.

20. An antioxidant of Claim 19, wherein said $(C_6-C_{12})$arylcarbonyl-$(C_1-C_5)$alkyl group is $-CH_2-CO-C_6H_5$.

21. An antioxidant of Claim 13, wherein said alkyl moiety in said group $R_2$ is a straight or branched chain.

22. An antioxidant of Claim 13, wherein said alkoxy moiety in said group $R_2$ consists of a straight or branched alkyl group and oxigen.

23. An antioxidant of Claim 13, wherein said alkenyl group is a straight or branched alkenyl group having at least one unsaturated double bond.

European Patent Office

# EUROPEAN SEARCH REPORT

Application Number

EP 89 11 4272

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 110, no. 6, 6th February 1989, page 373, abstract no. 44747t, Columbus, Ohio, US; & JP-A-63 190 882 (NIPPON HYPOX) | 1-23 | C 07 D 407/04<br>C 07 D 405/14<br>C 09 K 15/06<br>C 09 K 15/16<br>A 61 K 31/375 |
| Y | EP-A-0 202 589 (TAKEDA)<br>* The whole document, particularly pages 44,52,59,62 * | 1-23 | |
| Y | EP-A-0 146 121 (TAKEDA)<br>* The whole document * | 1-23 | |
| Y | CHEMICAL ABSTRACTS, vol. 106, no. 25, 22nd June 1987, page 701, abstract no. 214301c, Columbus, Ohio, US; & JP-A-61 236 772 (TAKEDA) | 1-23 | |
| X,Y | EP-A-0 086 554 (ELI LILLY)<br>* The whole document * | 1-23 | TECHNICAL FIELDS SEARCHED (Int. Cl.5) |
| Y | CARBOHYDRATE RESEARCH, vol. 102, 1982, pages 302-307, Amsterdam, NL; H.A. PARISH, Jr. et al.: "The reaction of 1,1-dibromopinacolone with L-ascorbic acid"<br>* The whole article, particularly page 303, compound 3 * | | C 07 D 407/00<br>C 07 D 405/00<br>C 09 K 15/00<br>A 61 K 31/00 |

The present search report has been drawn up for all claims

| Place of search | Date of completion of the search | Examiner |
|---|---|---|
| THE HAGUE | 30-03-1990 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)